# EUROPÄISCHE PATENTSCHRIFT

(11) **EP 0 301 484 B1**
(45) Veröffentlichungstag und Bekanntmachung des Hinweises auf die Patenterteilung: **22.06.1994**
(21) Anmeldenummer: 88112058.8
(22) Anmeldetag: 26.07.1988
(51) Int. Cl.: C12N 15/16, A61K 37/24

(54) **PTH-Modifikationen und therapeutische Zusammensetzungen, die sie enthalten und daraus bestehen**
PTH modifications and therapeutic compositions containing them or consisting of them
Modifications de PTH et compositions thérapeutiques les contenant ou composées d'elles

(30) Priorität: 30.07.1987 DE 3725319
(43) Veröffentlichungstag der Anmeldung: 01.02.1989
(73) Patentinhaber: Gesellschaft für Biotechnologische Forschung mbH (GBF), D-38124 Braunschweig (DE)
(72) Erfinder: Schlüter, Klaus-Dieter, D-3300 Braunschweig (DE); Mayer, Hubert, Dr., D-3300 Braunschweig (DE); Wingender, Edgar, Dr., D-3300 Braunschweig (DE)
(74) Vertreter: Boeters, Hans Dietrich, Dr.

(56) Entgegenhaltungen:
- WO-A-86/06742
- US-A- 4 423 037
- Born, W. et al. (1983), Experimenta 39, page 659
- JOURNAL OF BONE AND MINERAL RESEARCH, Band 1, Nr. 4, 1986, Mary Ann Liebert, Inc. Publishers; D.M. SLOVIK et al., S. 377-381

## Beschreibung

Parathormon (PTH) stimuliert als regulatorisches Hormon der Kalzium-Homöostasie eine Reihe von Enzymen, und zwar u. a. die Ornithindecarboxylase und die Adenylatcyclase. Zielorgane des PTH sind einmal die Niere und andererseits der Knochen. An der Niere fördert das Hormon die Resorption von Kalzium und am Knochen wirkt PTH bei der Mobilisierung von Kalzium mit.

In J. Cell. Biol., 24 (1965) 316-323 (1; Borle & Neumann) wird ein wachstumfördernder Effekt des PTH in vitro an Hela-Zellen, in Biochem. Biophys. Res. Comm., 66 (1975) 188-194 (2; Morgan et al.) ein derartiger Effekt an T-Lymphocyten und in Biochem. Biophys. Res. Comm., 129 (1985) 918-925 (3; van der Plas et al.) an Osteoblasten aus Hühnern beschrieben, wenn die Osteoblasten in Sekundärkultur gehalten wurden. In Endocrinology, 118 (1986) 2445-2449 (4; MacDonald et al.) wird ein derartiger Effekt an Knochenzellen beschrieben. Endocrinology, 110 (1982) 506-512 (5; Tam et al.) beschreibt eine gesteigerte in-vivo-Knochenanbaurate nach PTH-Zugabe, Endocr. Japan, 27 (1980) 349-356 (6; Kawashima et al.) und Calcif. Tissue Int., 35 (1983) 526-532 (7; Burch & Lebovitz) eine Stimulation der Proliferation durch PTH in vitro an Knorpelorgankulturen aus Hühnerembryonen und Calcif. Tissue Int., 38 (1986) 155-162 (8; Lewinson & Silbermann) an Knorpelorgankulturen aus Mäuseembryonen. Bei den genannten Arbeiten kamen jeweils PTH bzw. PTH-Fragmente zum Einsatz, die gleichzeitig zu einer Stimulierung der Adenylatcyclase an Nierencortexmembranen führten, so daß bei Verwendung dieser Substanzen als Material zur Wachstumsförderung des Knochens in vivo mit einer Beeinflussung der Nierenfunktion gerechnet werden mußte. Ferner konnten in den genannten Arbeiten keine definierten Zielzellen ermittelt werden oder wurden unspezifische und nicht am Wirkort "Knochen" lokalisierte Zellen als stimulierbar erkannt.

Bekannt ist auch, daß eine Deletion am natürlichen N-Terminus, wie sie durch das PTH-Fragment hPTH(28-48) belegt wird, dazu führt, daß die renale Adenylatcyclase nicht stimuliert wird; vgl. Adv. Protein Chemistry, 35 (1982) 323 ff. (10; Potts et al.). Es war danach nicht zu erwarten, daß bei einer PTH-Deletion die mitogene Wirkung nicht gleichfalls verlorengeht.

Aufgabe der Erfindung ist es, pPTH-, bPTH- oder hPTH-Modifikationen sowie therapeutische Zusammensetzungen mit diesen Modifikationen vorzusehen, welche die renale Adenylatcyclase nicht stimulieren, sondern vielmehr eine Chondrocyten-Proliferation bewirken, also einen mitogenen bzw. knochenspezifischen Effekt zeigen.

Diese Aufgabe wird durch die Gegenstände der Ansprüche gelöst.

Zur Herstellung von PTH-Modifikationen, die N-terminal verlängert sind, wird auf die DE-A-33 12 928 sowie auf die EP 88 112 059.6 ..... verwiesen. Deletierte PTH-Modifikationen sind im Handel erhältlich, aus der US-A-4 423 037 bekannt, oder lassen sich aus PTH nach geläufigen Peptid-Abbaumethoden gewinnen.

Nachstehend wird die Erfindung beispielhaft näher erläutert. Es zeigen:

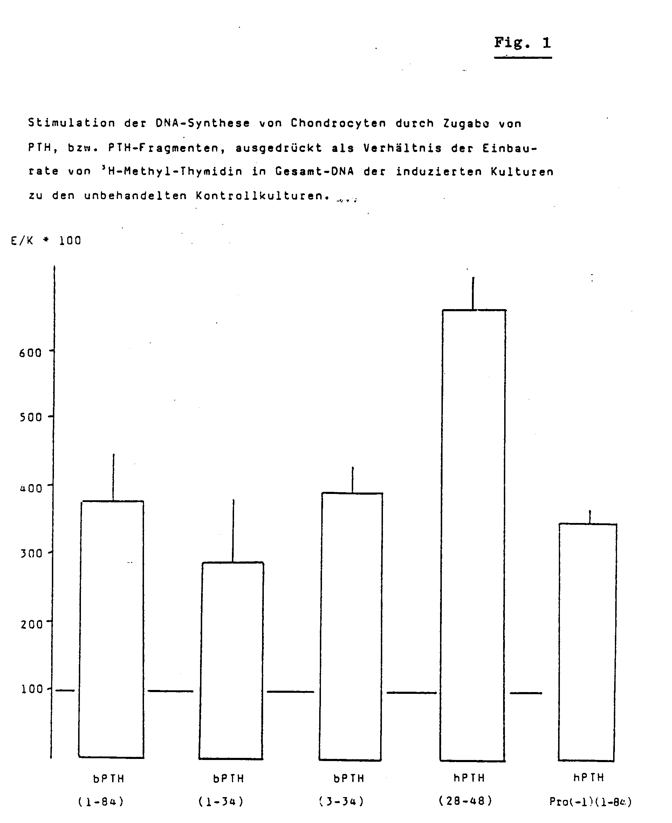

Fig. 1 eine graphische Darstellung der Stimulation der DNA-Synthese von Chondrocyten durch Zugabe von PTH bzw. PTH-Fragmenten;

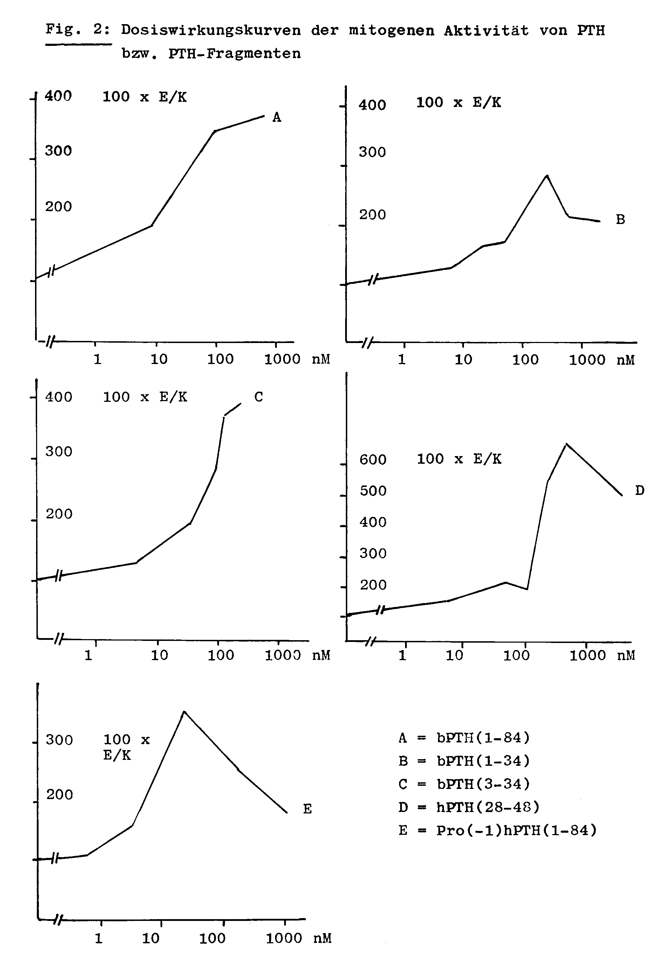

Fig. 2 eine graphische Darstellung der Dosis/Wirkungs-Kurven der mitogenen Aktivität von PTH bzw. PTH-Fragmenten; und

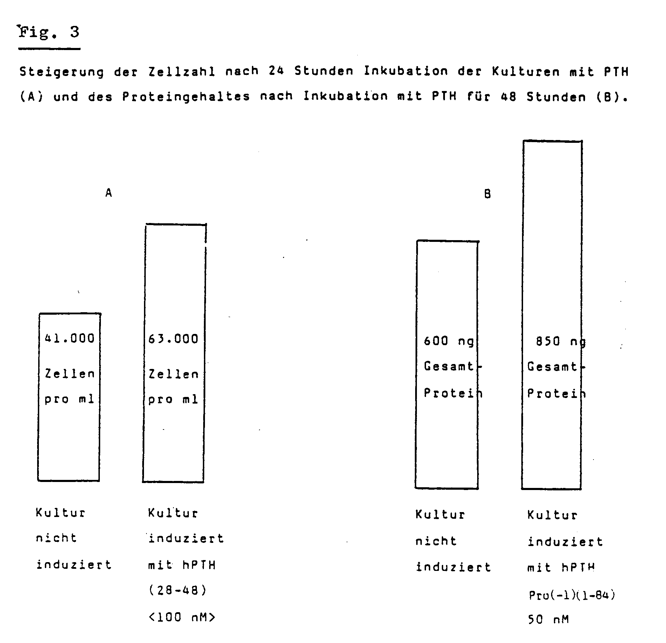

Fig. 3 eine graphische Darstellung für Zellzahl und Gesamtprotein nach Stimulation mit PTH.

### Figur 1:

Die erfindungsgemäße Wirkung wurde an Chondrocyten gezeigt, die durch fraktionierte Collagenase-Behandlung aus den Sterna 16 Tage alter Hühnerembryonen gemäß der in J. Biol. Chem., 261 (1986) 7997-8001 (9; Yasui et al.) beschriebenen Methode isoliert wurden. Und zwar wurde der wachstumstimulierende Effekt durch Einbau von ³H-Methylthymidin in Gesamt-DNA, Zellzahlbestimmung und Bestimmung des Gesamtproteingehaltes der Kulturen gezeigt. Zur Bestimmung des Einbaus des ³H-Methylthymidins in Gesamt-DNA wurden die Zellen auf Mikrotiterplatten in M199-Medium bei einer Konzentration von fötalem Kälberserum (FCS) kultiviert, die kleiner 0,5 % (Volumenbasis) war. Die Zelldichte der Chondrocyten, die mit einer Steigerung des Einbaus von ³H-Methylthymidin in die GesamtDNA auf die PTH-Zugabe reagierten, lag bei 1250 bis 6000 Zellen/Mikrotiterloch (Durchmesser 6 mm) im Zeitpunkt des Ausplattierens. Eine maximale Antwort wurde bei einer Zelldichte von 3000 Zellen/Mikrotiterloch (Durchmesser 6 mm) im Zeitpunkt des Ausplattierens innerhalb von 4 h nach PTH-Zugabe und nach einem 4 bis 10 Tage langen Wachstum der Chondrocyten ohne Mediumwechsel erreicht. Der ³H-Methylthymidin-Einbau in die Gesamt-DNA wurde folgendermaßen bestimmt. Die adhärent wachsenden Chondrocyten wurden nach Entfernen des radioaktiven Mediums mit PBS gewaschen und in den Mikrotiterlöchern lysiert. Danach wurde die GesamtDNA sauer und kalt gefällt und mit Hilfe eines semi-automatischen Zellerntegerätes auf Filterpapiere überführt. Danach konnte die Einbaurate in Gesamt-DNA bestimmt werden.

### Figur 2:

bPTH(1-84), bPTH(1-34) und bPTH(3-34) sowie Pro(-1)hPTH(1-84) zeigten eine signifikante Zunahme des ³H-Methylthymidin-Einbaus in Gesamt-DNA um einen Faktor im Bereich von 3,0 bis 3,5, und zwar in Abhängigkeit von der Konzentration an zugegebenem PTH bzw. zugegebenen PTH-Fragmenten. Die Konzentration für eine halbmaximale Stimulation lag für bPTH(1-84) und Pro(-1)hPTH(1-84) bei 9 bis 15 nM und für die PTH-Fragmente bPTH(1-34) und bPTH(3-34) bei 50 bis 75 nM. Hingegen zeigte das PTH-Fragment hPTH(28-48), das nach Adv. in Protein Chemistry, 35 (1982) 323 ff. (10; Potts et al.) die renale Adenylatcyclase nicht stimuliert und nicht am renalen PTH-Rezeptor bindet, bei einer Konzentration für halbmaximale Stimulation von 349 nM einen gesteigerten Einbau um den Faktor 6. Hohe Konzentrationen an PTH bzw. PTH-Fragmenten führten dagegen nicht zu einer Stimulation des Chondrocytenwachstums. PTH oder PTH-Fragmente zeigten in den genannten Konzentrationen keinen wachstumsstimulatorischen Effekt auf Osteoblast-Vorläuferzellen.

### Figur 3:

hPTH(28-48) führte ferner zu einer Steigerung der Gesamtzellzahl von 41.000 Zellen/ml auf 63.000 Zellen/ml. Pro(-1)hPTH(1-84) führte zu einer Steigerung des Gesamtproteingehalts von 600 ng Protein/ml auf 850 ng Protein/ml.

## Patentansprüche

1. Verwendung einer pPTH-, bPTH- oder hPTH-Modifikation, bei der der natürliche N-Terminus und der natürliche C-Terminus außerhalb des Aminosäurebereichs von +28 bis +34 deletiert ist, zur Herstellung einer therapeutischen Zusammensetzung mit mitogenem Effekt.

2. Verwendung einer pPTH-, bPTH- oder hPTH-Modifikation, bei der der natürliche N-Terminus und der natürliche C-Terminus außerhalb des Aminosäurebereichs von +28 bis +48 deletiert ist, zur Herstellung einer therapeutischen Zusammensetzung mit mitogenem Effekt.

3. Verwendung von bPTH (3-34) und/oder hPTH (28-48) zur Herstellung einer therapeutischen Zusammensetzung mit mitogenem Effekt.

## Claims

1. The use of a pPTH, bPTH or hPTH modification, in which the natural N-terminus and the natural C-terminus has been deleted beyond the amino acid region of from +28 to +34, for the preparation of a therapeutic composition having a mitogenic effect.

2. The use of a pPTH, bPTH or hPTH modification, in which the natural N-terminus and the natural C-terminus has been deleted beyond the amino acid region of from +28 to +48, for the preparation of a therapeutic composition having a mitogenic effect.

3. The use of bPTH (3-34) and/or hPTH (28-48) for the preparation of a therapeutic composition having a mitogenic effect.

## Revendications

1. Utilisation de pPTH, bPTH ou hPTH modifiées, dans lesquelles l'extrémité N-terminale naturelle et l'extrémité C-terminale naturelle sont délétées à l'extérieur de la région d'acides aminés allant de +28 à +34, pour la préparation d'une composition thérapeutique ayant un effet mitogène.

2. Utilisation de pPTH, bPTH ou hPTH modifiées, dans lesquelles l'extrémité N-terminale naturelle et l'extrémité C-terminale naturelle sont délétées à l'extérieur de la région d'acides aminés allant de +28 à +48, pour la préparation d'une composition thérapeutique ayant un effet mitogène.

3. Utilisation de bPTH (3-34) et/ou de hPTH (28-48), pour la préparation d'une composition thérapeutique ayant un effet mitogène.
